# EUROPEAN PATENT SPECIFICATION

(11) **EP 4 197 451 B1**
(45) Date of publication and mention of the grant of the patent: **12.08.2026**
(21) Application number: 22213993.3
(22) Date of filing: 15.12.2022
(51) Int. Cl.: A61B 10/00, G01N 21/75, G01N 21/84

(54) **REPLACEMENT BOX FOR URINALYSIS DEVICE, REPLACEMENT BOX BOTTOM SUPPORT, AND REPLACEMENT BOX MOUNTING STRUCTURE**
AUSTAUSCHKASTEN FÜR EINE URINANALYSEVORRICHTUNG, ERSATZKASTENBODENSTÜTZE UND AUSTAUSCHKASTENMONTAGESTRUKTUR
BOÎTE DE REMPLACEMENT POUR DISPOSITIF D'ANALYSE D'URINE, SUPPORT INFÉRIEUR DE BOÎTE DE REMPLACEMENT ET STRUCTURE DE MONTAGE DE BOÎTE DE REMPLACEMENT

(30) Priority: 17.12.2021 CN 202123196255 U; 08.12.2022 US 202218077626
(43) Date of publication of application: 21.06.2023
(73) Proprietor: Shanghai Kohler Electronics, Ltd., Shanghai 200444 (CN)
(72) Inventor: WANG, Zhenwei, Shanghai 200438 (CN)
(74) Representative: Barker Brettell LLP

(56) References cited:
- WO-A1-2020/139577
- WO-A2-2021/175909
- CN-A- 110 101 329
- CN-U- 205 894 230
- US-A1- 2021 386 409

## Description

### CROSS REFERENCE TO RELATED APPLICATION

This application claims the benefit of priority to Chinese Patent Application No. 202123196255.4 filed in the Chinese Intellectual Property Office on December 17, 2021, and US Patent Application No. 18/077,626 filed in the US Patent & Trademark Office on December 8, 2022.

### FIELD

The present disclosure relates to the field of technologies related to a sanitary equipment, in particular to a replacement box for a urinalysis device, a replacement box bottom support, and a replacement box mounting structure.

### BACKGROUND

A urinalysis device may automatically use test paper for physical fitness testing. However, the test paper needs to be replaced by a customer after the test paper is used up.

However, when the customer manually mounts a replacement, it is difficult to detect whether the replacement is mounted or installed in place only by feeling or intuition. When the replacement is not mounted or installed in place, it is easy to cause a system action error.

Therefore, a position switch or an optocoupler switch is used to detect whether the replacement is in place. However, this detection method has a larger stroke and a lower stroke precision.

WO 2021/175909 A2 is considered the closest prior art, and discloses a urinalysis device that can be attached to the interior of a toilet bowl using a combination of a suction cup device and magnets comprised in the suction cup device and the housing of the urinalysis device.

US 2021/386409 A1 discloses a magnetically mountable sensor for a health care mirror.

### SUMMARY

In view of the above technical problem, it is necessary to provide a replacement box for a urinalysis device, a replacement box bottom support, and a replacement box mounting structure to address the technical problem that the urinalysis device cannot effectively ensure in-place mounting or installation of a replacement for a urinalysis device.

The invention is defined in independent claim 1. Independent claim 8 defines a method of operating the inventive device. Further embodiments are defined in the dependent claims. The present disclosure provides a replacement box mounting structure for a a box body cavity, test paper is contained or housed in the box body cavity, and a bottom portion of the box body lower cover is provided with a first magnetic attraction device.

In an embodiment, the first magnetic attraction device is a magnet or a magnetic urinalysis device as defined in the appended claims. Optional features are defined in the dependent claims.

According to the present disclosure, the replacement box is guided and positioned to a preset position through the first magnetic attraction device and the second magnetic attraction device, and detection is conducted through the detection switch. Thus, a guiding effect is provided for a user to mount the replacement box, and an in-place detection precision is improved.

### BRIEF DESCRIPTION OF THE FIGURES

FIG. 1 is a schematic structural diagram of a replacement box for a urinalysis device according to an example of the present disclosure;
FIG. 2 is a schematic structural diagram of a replacement box bottom support for the urinalysis device according to an example of the present disclosure;
FIG. 3 is a top view of the replacement box bottom support for the urinalysis device according to an example of the present disclosure;
FIG. 4 is a sectional view of a replacement box mounting structure for the urinalysis device according to an example of the present disclosure; and
FIG. 5 is a flow chart of a method for assembling the replacement box mounting structure for the urinalysis device according to an example of the present disclosure.

### Descriptions of reference numerals

1 refers to a replacement box; 11 refers to a box body upper cover; 111 refers to an upper cover bottom surface; 12 refers to a box body lower cover; 13 refers to a box body cavity; 14 refers to test paper; 15 refers to a first magnetic attraction device; 2 refers to a replacement box bottom support; 21 refers to a bottom support body; 22 refers to a bottom support cavity; 23 refers to a second magnetic attraction device; and 24 refers to a detection switch.

### DETAILED DESCRIPTION

Specific embodiments of the present disclosure are further described hereinafter with reference to the drawings. The same or equivalent parts are denoted by the same reference numerals. It should be noted that the terms "front", "back", "left", "right", "up" and "down" used in the following description refer to the directions in the drawings, and the terms "inner" and "outer" refer to the directions towards or far away from geometric centers of specific parts respectively.

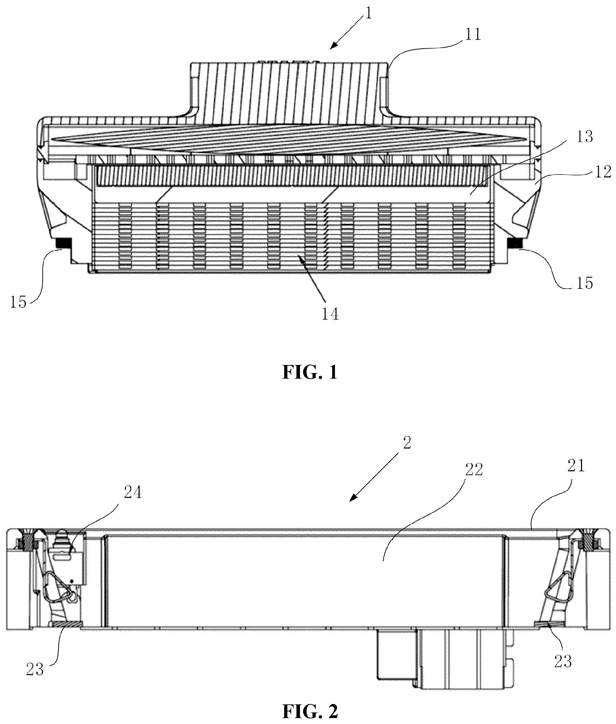

FIG. 1 is a schematic structural diagram of a replacement box 1 for a urinalysis device of the present disclosure. The replacement box for the urinalysis device comprises a box body upper cover 11 and a box body lower cover 12. The box body upper cover 11 and the box body lower cover 12 are connected to form a box body cavity 13. Test paper 14 is contained in the box body cavity 13. A bottom portion of the box body lower cover 12 is provided with a first magnetic attraction device 15.

Specifically, the replacement box 1 is replaced in the urinalysis device as a whole. The box body upper cover 11 and the box body lower cover 12 are connected to form the box body cavity 13 for containing the test paper 14. In an embodiment, the test paper 14 may be placed in the box body lower cover 12. Finally, the first magnetic attraction device 15 is installed on the bottom portion of the box body lower cover 12.

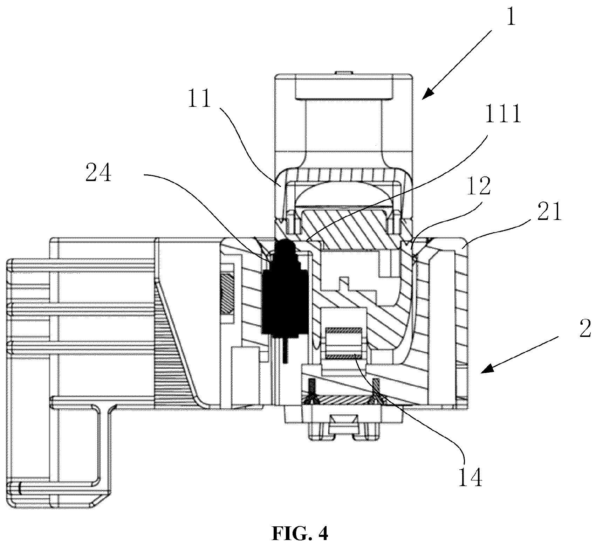

FIG. 4 is a sectional view of a replacement box mounting structure for the urinalysis device according to an example of the present disclosure. As shown in FIG. 4, when in use, the replacement box 1 is inserted into a replacement box bottom support 2 for the urinalysis device. A bottom portion of the replacement box bottom support 2 is provided with a second magnetic attraction device 23. The first magnetic attraction device 15 and the second magnetic attraction device 23 are attracted to each other, so that the replacement box 1 can be guided to reach a preset position.

According to the present disclosure, the replacement box is guided and positioned to the preset position through the first magnetic attraction device and the second magnetic attraction device. An in-place detection regarding whether the replacement box reaches the preset position is conducted through a detection switch. Thus, a guiding effect is provided for a user to mount or install the replacement box, and the in-place detection precision can be improved.

FIG. 1 shows a replacement box 1 for a urinalysis device in an embodiment of the present disclosure, The replacement box for the urinalysis device comprises a box body upper cover 11 and a box body lower cover 12. The box body upper cover 11 and the box body lower cover 12 are connected to form a box body cavity 13, test paper 14 is contained or housed in the box body cavity 13. A bottom portion of the box body lower cover 12 is provided with a first magnetic attraction device 15. The first magnetic attraction device 15 is a magnet or a magnetic attraction iron sheet. An upper cover bottom surface 111 of the box body upper cover 11 protrudes beyond the box body lower cover 12.

Specifically, the first magnetic attraction device 15 may be the magnet or the magnetic attraction iron sheet. When the first magnetic attraction device 15 is a magnet, a second magnetic attraction device 23 is a magnetic attraction iron sheet. When the first magnetic attraction device 15 is a magnetic attraction iron sheet, the second magnetic attraction device 23 is a magnet. The magnet may be replaced by any magnetic materials. The magnetic attraction iron sheet may be replaced by any materials that may be magnetically attracted to the magnet. The first magnetic attraction device 15 and the second magnetic attraction device 23 are attracted to each other, so that the replacement box 1 can be guided to reach a preset position.

As shown in FIG. 4, the upper cover bottom surface 111 of the box body upper cover 11 protrudes beyond the box body lower cover 12. When the replacement box 1 is inserted into a replacement box bottom support 2, the box body lower cover 12 is inserted into a bottom support cavity 22, and a protruding part or portion of the upper cover bottom surface 111 of the box body upper cover 11 may trigger a detection switch 24 of the replacement box bottom support 2, e.g., by pressing the detection switch 24, so that an in-place signal can be generated.

In the embodiment, the attraction is realized by using the magnet or the magnetic attraction iron sheet, the replacement box is guided and positioned to a preset position, and the detection switch is triggered by the box body upper cover to generate the in-place signal, so that an in-place detection precision can be improved.

FIG. 2 is a schematic structural diagram of a replacement box bottom support 2 for a urinalysis device of the present disclosure. The replacement box bottom support for the urinalysis device comprises a bottom support body 21. A bottom support cavity 22 for containing a replacement box is arranged in the bottom support body 21, a bottom portion of the bottom support cavity 22 is provided with a second magnetic attraction device 23, and a detection switch 24 is also fixed on the bottom support body 21.

Specifically, the replacement box bottom support 2 is used for containing the replacement box 1. The bottom support cavity 22 is arranged in the bottom support body 21, and the replacement box 1 is inserted into the bottom support cavity 22. The bottom portion of the bottom support cavity 22 is provided with the second magnetic attraction device 23.

As shown in FIG. 4, when in use, the replacement box 1 is inserted into the bottom support cavity 22. A bottom portion of the replacement box 1 is provided with a first magnetic attraction device 15. The first magnetic attraction device 15 and the second magnetic attraction device 23 are attracted to each other, so that the replacement box 1 can be guided to reach a preset position.

According to the present disclosure, the replacement box is guided and positioned to the preset position through the first magnetic attraction device and the second magnetic attraction device. An in-place detection regarding whether the replacement box reaches the preset position is conducted through the detection switch. Thus, a guiding effect is provided for a user to mount or install the replacement box, and the in-place detection precision can be improved.

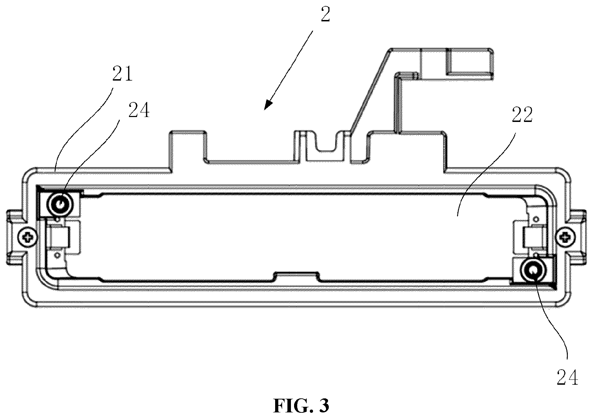

FIG. 2 and FIG. 3 show a replacement box bottom support 2 for a urinalysis device in an embodiment of the present disclosure. The replacement box bottom support for the urinalysis device comprises a bottom support body 21. A bottom support cavity 22 for containing a replacement box is arranged in the bottom support body 21. A bottom portion of the bottom support cavity 22 is provided with a second magnetic attraction device 23. A detection switch 24 and an indicating device in communication connection with the detection switch 24 are also fixed on the bottom support body 21. The second magnetic attraction device 23 is a magnetic attraction iron sheet or a magnet.

Specifically, a first magnetic attraction device 15 may be a magnet or a magnetic attraction iron sheet. When the first magnetic attraction device 15 is a magnet, the second magnetic attraction device 23 is a magnetic attraction iron sheet. When the first magnetic attraction device 15 is a magnetic attraction iron sheet, the second magnetic attraction device 23 is a magnet. The magnet may be replaced by any magnetic materials. The magnetic attraction iron sheet may be replaced by any materials that may be magnetically attracted to the magnet. The first magnetic attraction device 15 and the second magnetic attraction device 23 are attracted to each other, so that the replacement box 1 is guided to reach a preset position.

In an embodiment, two detection switches 24 may be respectively mounted on two sides of the bottom support body 21. The detection switch 24 may be a push switch, especially a high-precision switch. The detection switch 24 is in a free release state when there is no pressure applied to the detection switch 24. When the replacement box 1 is inserted into the replacement box bottom support 2, a box body lower cover 12 is inserted into the bottom support cavity 22, and a protruding part or portion of an upper cover bottom surface 111 of a box body upper cover 11 may trigger the detection switch 24 of the replacement box bottom support 2, e.g., by pressing the detection switch 24, so that an in-place signal can be generated.

The indicating device may be an indicator light, the indicating device is communicably coupled to the detection switch 24, and the indicating device, e.g., the indicator light, is turned on according to the in-place signal of the detection switch 24, so as to indicate that an in-place mounting is achieved, i.e., the replacement box 1 reaches the preset position.

In the embodiment, the attraction is realized by using the magnet or the magnetic attraction iron sheet, such that the replacement box can be guided and positioned to the preset position. Also, the in-place signal is generated through the detection switch, and whether in-place mounting is realized is indicated to a customer through the indicating device.

FIG. 4 is a sectional view of a replacement box mounting structure for a urinalysis device of the present disclosure. The replacement box mounting structure for the urinalysis device comprises the replacement box 1 as described above and the replacement box bottom support 2 as described above. The replacement box 1 is inserted into the replacement box bottom support 2 along a first direction. The first magnetic attraction device 15 and the second magnetic attraction device 23 are at least partially overlapped along the first direction when the replacement box 1 is inserted into the replacement box bottom support 2.

Specifically, when in use, the replacement box 1 is inserted into the replacement box bottom support 2 for the urinalysis device, the bottom portion of the replacement box 1 is provided with the first magnetic attraction device 15, and a bottom portion of the replacement box bottom support 2 is provided with the second magnetic attraction device 23. The first magnetic attraction device 15 and the second magnetic attraction device 23 are at least partially overlapped along the first direction, so that the first magnetic attraction device 15 and the second magnetic attraction device 23 can be attracted to each other and the replacement box 1 can be guided to reach a preset position.

According to the present disclosure, the replacement box is guided and positioned to the preset position through the first magnetic attraction device and the second magnetic attraction device. An in-place detection regarding whether the replacement box reaches the preset position is conducted through the detection switch. Thus, a guiding effect is provided for a user to mount or install the replacement box, and the in-place detection precision is improved.

FIG. 4 is a sectional view of a replacement box mounting structure for a urinalysis device of the present disclosure. The replacement box mounting structure for the urinalysis device comprises the replacement box 1 as described above and the replacement box bottom support 2 as described above. The replacement box 1 is inserted into the replacement box bottom support 2 along a first direction. The first magnetic attraction device 15 and the second magnetic attraction device 23 are at least partially overlapped along the first direction when the replacement box 1 is inserted into the replacement box bottom support 2.

The first magnetic attraction device 15 is a magnet and the second magnetic attraction device 23 is a magnetic attraction iron sheet; or the first magnetic attraction device 15 is a magnetic attraction iron sheet and the second magnetic attraction device 23 is a magnet. The magnet may be replaced by any magnetic materials. The magnetic attraction iron sheet may be replaced by any materials that may be magnetically attracted to the magnet.

A height from the upper cover bottom surface 111 of the box body upper cover 11 protruding beyond the box body lower cover 12 to a bottom portion of the first magnetic attraction device 15 is smaller than a height from a top portion of the second magnetic attraction device 23 to a top portion of the detection switch 24. When the box body lower cover 12 is inserted into the bottom support cavity 22, the box body upper cover 11 presses the detection switch 24.

Specifically, the detection switch 24 may be a push switch. The height from the upper cover bottom surface 111 of the box body upper cover 11 protruding beyond the box body lower cover 12 to the bottom portion of the first magnetic attraction device 15 is smaller than the height from the top portion of the second magnetic attraction device 23 to the top portion of the detection switch 24. When the replacement box 1 is inserted into the replacement box bottom support 2, the box body lower cover 12 is inserted into the bottom support cavity 22, so that a protruding part of the upper cover bottom surface 111 of the box body upper cover 11 can trigger the detection switch 24 of the replacement box bottom support 2, e.g., by pressing the detection switch 24, to generate an in-place signal.

In the embodiment, the attraction is realized by using the magnet or the magnetic attraction iron sheet, so that the replacement box can be guided and positioned to a preset position. Also, the detection switch is triggered by the box body upper cover to generate the in-place signal, so that an in-place detection precision can be improved.

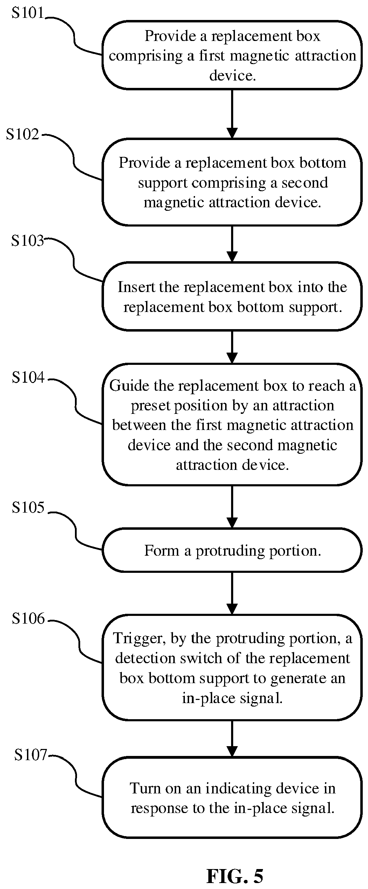

FIG. 5 is a flow chart of a method for assembling a replacement box mounting structure for a urinalysis device according to an example of the present disclosure. The replacement box mounting structure to be assembled by the method may be the replacement box mounting structure according to any of the foregoing embodiments. The replacement box and the replacement box bottom support of the replacement box mounting structure are described above in the present disclosure. The replacement box, the replacement box bottom support, and the replacement box mounting structure are configured to perform an operation, function, or the like as described above in the present disclosure.

At act S101, a user may provide the replacement box 1 comprising the first magnetic attraction device 15. The first magnetic attraction device 15 is disposed at the bottom portion of the box body lower cover 12 of the replacement box 1.

At act S102, the user may provide the replacement box bottom support 2 comprising the second magnetic attraction device. The second magnetic attraction device 23 is disposed at the bottom portion of the bottom support cavity 22.

At act S103, the user may insert the replacement box 1 into the bottom support cavity 22 of the replacement box bottom support 2. The direction along which the replacement box 1 into the bottom support cavity 22 refer to the "first direction" in the present disclosure.

At act S104, an attraction between the first magnetic attraction device 15 and the second magnetic attraction device 23 may guide the replacement box 1 to reach a preset position. As described above, when the first magnetic attraction device 15 is a magnet, the second magnetic attraction device 23 is a magnetic attraction iron sheet. When the first magnetic attraction device 15 is a magnetic attraction iron sheet, the second magnetic attraction device 23 is a magnet. The magnet may be replaced by any magnetic materials. The magnetic attraction iron sheet may be replaced by any materials that may be magnetically attracted to the magnet. The first magnetic attraction device 15 and the second magnetic attraction device 23 are attracted to each other, so that the replacement box 1 can be guided to reach the preset position.

At act S105, the user may form the protruding portion by allowing the upper cover bottom surface 111 of the box body upper cover 11 of the replacement box 1 to protrude beyond the box body lower cover 12 of the replacement box 1.

At act S106, the protruding portion may trigger the detection switch 24 of the replacement box bottom support 2 to generate the in-place signal. Specifically, when the replacement box 1 reaches the preset position, the protruding portion may press the detection switch 24 and thus trigger the detection switch 24 to generate the in-place signal.

In another embodiment, two detection switches 24 may be respectively disposed on two sides of the bottom support body 21. When the replacement box 1 is inserted into the bottom support cavity 22 of the replacement box bottom support 2, the in-place signal may be generated when the two detection switches 24 are both triggered by the protruding portion.

At act S107, the in-place signal generated by the detection switch 24 may turn on the indicating device. The indicating device is communicably coupled to the at least one detection switch. As described above, the indicating device may be an indicator light configured to indicate that an in-place mounting is achieved, i.e., the replacement box 1 reaches the preset position.

The above embodiments merely express several embodiments of the present disclosure, and the descriptions thereof are more specific and detailed. However, it should not be understood as a limitation to the patent scope of the present disclosure. It should be noted that those of ordinary skill in the art may make a plurality of variations and improvements without departing from the conception of the present disclosure. These variations and improvements shall all fall within the protection scope of the present disclosure. Therefore, the protection scope of the patent according to the present disclosure shall be subjected to the appended claims.

An aspect provides a replacement box for a urinalysis device, comprising a box body upper cover and a box body lower cover, wherein the box body upper cover and the box body lower cover are connected to form a box body cavity, test paper is contained in the box body cavity, and a bottom portion of the box body lower cover is provided with a first magnetic attraction device.

The first magnetic attraction device may comprise a magnet or a magnetic attraction iron sheet.

An upper cover bottom surface of the box body upper cover protrudes beyond the box body lower cover.

The replacement box bottom support for a urinalysis device comprises a bottom support body, wherein a bottom support cavity for containing a replacement box is arranged in the bottom support body, a bottom portion of the bottom support cavity is provided with a second magnetic attraction device, and a detection switch is also fixed on the bottom support body. The second magnetic attraction device may comprise a magnetic attraction iron sheet or a magnet.

The replacement box bottom support may comprise an indicating device in communication connection with the detection switch.

In an implementation, the replacement box is inserted into the replacement box bottom support along a first direction, and the first magnetic attraction device and the second magnetic attraction device are at least partially overlapped along the first direction.

In an implementation, the first magnetic attraction device is a magnet and the second magnetic attraction device is a magnetic attraction iron sheet; or the first magnetic attraction device is a magnetic attraction iron sheet and the second magnetic attraction device is a magnet.

In an implementation, the box body lower cover is inserted into the bottom support cavity, and the box body upper cover presses the detection switch.

In an implementation, a height from the upper cover bottom surface of the box body upper cover protruding beyond the box body lower cover to a bottom portion of the first magnetic attraction device is smaller than a height from a top portion of the second magnetic attraction device to a top portion of the detection switch.

## Claims

1. A replacement box (1) mounting structure for a urinalysis device comprising:
a replacement box (1) comprising:
a box body upper cover (11);
a box body lower cover (12) configured to be connected to the box body upper cover (11) to form a box body cavity (13), the box body cavity (13) configured to house test paper (14); and
a first magnetic attraction device (15) disposed at a bottom portion of the box body lower cover (12); and
a replacement box bottom support (2) comprising:
a bottom support body (21); characerised by
at least one detection switch (24) disposed at the bottom support body (21) and configured to detect whether the replacement box (1) reaches a preset position;
a bottom support cavity (22) disposed in the bottom support body (21) and configured to receive the replacement box (1); and
a second magnetic attraction device (23) disposed at a bottom portion of the bottom support cavity (22),
wherein the first magnetic attraction device (15) is configured to be attracted to guide the replacement box (1) to reach the preset position when the replacement box (1) is inserted into the replacement box bottom support (2),
wherein the second magnetic attraction device (23) of the replacement box bottom support (2) is configured to guide the replacement box (1) to reach the preset position when the replacement box (1) is inserted into the replacement box bottom support (2),
wherein an upper cover bottom surface (111) of the box body upper cover (11) protrudes beyond the box body lower cover (12) to form a protruding portion, and
wherein the protruding portion of the box body upper cover (11) is configured to trigger the at least one detection switch (24) of the replacement box bottom support (2) so as to generate an in-place signal by pressing the at least one detection switch (24).

2. The replacement box (1) mounting structure according to claim 1, wherein the first magnetic attraction device (15) is configured to be attracted to a second magnetic attraction device (23) of the replacement box bottom support (2) so as to reach the preset position when the replacement box (1) is inserted into the replacement box bottom support (2).

3. The replacement box (1) mounting structure according to claim 2, wherein the first magnetic attraction device (15) is made of a magnetic material or a metal material.

4. The replacement box (1) according to claim 3, wherein one of the first magnetic attraction device (15) and the second magnetic attraction device (23) is made of the magnetic material and the other of the first magnetic attraction device (15) and the second magnetic attraction device (23) is made of the metal material

5. The replacement box (1) mounting structure according to any one of the preceding claims, wherein:
(i) the in-place signal is configured to turn on an indicating device of the replacement box bottom support (2) and the indicating device is communicably coupled to the at least one detection switch (24); and/or
(ii) the protruding portion of the box body upper cover (11) is configured to trigger two detection switches (24) of the replacement box bottom support (2) and the two detection switches (24) are respectively disposed on two sides of the replacement box bottom support (2).

6. The replacement box (1) mounting structure according to any one of the preceding claims, wherein the second magnetic attraction device (23) of the replacement box bottom support (2) is made of a metal material or a magnetic material.

7. The replacement box (1) mounting structure according to any one of the preceding claims, wherein a height from the upper cover bottom surface (111) of the box body upper cover (11) protruding beyond the box body lower cover (12) to a bottom portion of the first magnetic attraction device (15) of the replacement box (1) is smaller than a height from a top portion of the second magnetic attraction device (23) of the replacement box bottom support (2) to a top portion of the at least one detection switch (24).

8. A method for assembling a replacement box (1) mounting structure for a urinalysis device, the method comprising:
providing a replacement box (1) comprising a first magnetic attraction device (15) disposed at a bottom portion of a box body lower cover (12) of the replacement box (1) and comprising a protruding portion by allowing an upper cover bottom surface (111) of a box body upper cover (11) of the replacement box to protrude beyond the box body lower cover (12) of the replacement box (1);
providing a replacement box bottom support (2) comprising a second magnetic attraction device (23) disposed at a bottom portion of a bottom support cavity (22);
inserting the replacement box (1) into the bottom support cavity (22) of the replacement box bottom support (2); and
guiding the replacement box (1) to reach a preset position by an attraction between the first magnetic attraction device (15) and the second magnetic attraction device (23); and
triggering, by the protruding portion, at least one detection switch (24) of the replacement box bottom support (2) to generate an in-place signal.

9. The method according to claim 10, further comprising:
turning on an indicating device, communicably coupled to the at least one detection switch (24), in response to the in-place signal generated by the at least one detection switch (24).

## Patentansprüche

1. Austauschkasten-(1)Montagestruktur für eine Urinanalysevorrichtung, umfassend:
einen Austauschkasten (1), umfassend:
eine obere Abdeckung (11) des Kastenkörpers;
eine untere Abdeckung (12) des Kastenkörpers, die dazu konfiguriert ist, mit der oberen Abdeckung (11) des Kastenkörpers verbunden zu werden, um einen Kastenkörperhohlraum (13) zu bilden, wobei der Kastenkörperhohlraum (13) dazu konfiguriert ist, Prüfpapier (14) aufzunehmen; und
eine erste magnetische Anziehungsvorrichtung (15), die an einem Bodenabschnitt der unteren Abdeckung (12) des Kastenkörpers angeordnet ist; und
eine Austauschkastenbodenstütze (2), die Folgendes umfasst:
einen Bodenstützenkörper (21); charakterisiert durch
mindestens einen Erfassungsschalter (24), der an dem Bodenstützenkörper (21) angeordnet und dazu konfiguriert ist, zu erfassen, ob der Austauschkasten (1) eine voreingestellte Position erreicht;
einen Bodenstützenhohlraum (22), der in dem Bodenstützenkörper (21) angeordnet und dazu konfiguriert ist, den Austauschkasten (1) zu empfangen; und
eine zweite magnetische Anziehungsvorrichtung (23), die an einem Bodenabschnitt des Bodenstützenhohlraums (22) angeordnet ist,
wobei die erste magnetische Anziehungsvorrichtung (15) dazu konfiguriert ist, angezogen zu werden, um den Austauschkasten (1) zu führen, um die voreingestellte Position zu erreichen, wenn der Austauschkasten (1) in die Austauschkastenbodenstütze (2) eingesetzt wird,
wobei die zweite magnetische Anziehungsvorrichtung (23) der Austauschkastenbodenstütze (2) dazu konfiguriert ist, den Austauschkasten (1) zu führen, um die voreingestellte Position zu erreichen, wenn der Austauschkasten (1) in die Austauschkastenbodenstütze (2) eingesetzt wird,
wobei eine Bodenfläche (111) einer oberen Abdeckung der oberen Abdeckung (11) des Kastenkörpers über die untere Abdeckung (12) des Kastenkörpers hinausragt, um einen hinausragenden Abschnitt zu bilden, und
wobei der hinausragende Abschnitt der oberen Abdeckung (11) des Kastenkörpers dazu konfiguriert ist, den mindestens einen Erfassungsschalter (24) der Austauschkastenbodenstütze (2) auszulösen, um ein Signal an Ort und Stelle durch Drücken des mindestens einen Erfassungsschalters (24) zu erzeugen.

2. Austauschkasten-(1)Montagestruktur nach Anspruch 1, wobei die erste magnetische Anziehungsvorrichtung (15) dazu konfiguriert ist, zu einer zweiten magnetischen Anziehungsvorrichtung (23) der Austauschkastenbodenstütze (2) angezogen zu werden, um die voreingestellte Position zu erreichen, wenn der Austauschkasten (1) in die Austauschkastenbodenstütze (2) eingesetzt wird.

3. Austauschkasten-(1)Montagestruktur nach Anspruch 2, wobei die erste magnetische Anziehungsvorrichtung (15) aus einem magnetischen Material oder einem Metallmaterial hergestellt ist.

4. Austauschkasten (1) nach Anspruch 3, wobei eine der ersten magnetischen Anziehungsvorrichtung (15) und der zweiten magnetischen Anziehungsvorrichtung (23) aus dem magnetischen Material hergestellt ist und die andere der ersten magnetischen Anziehungsvorrichtung (15) und der zweiten magnetischen Anziehungsvorrichtung (23) aus dem Metallmaterial hergestellt ist.

5. Austauschkasten-(1)Montagestruktur nach einem der vorhergehenden Ansprüche, wobei:
(i) das Signal an Ort und Stelle dazu konfiguriert ist, eine Anzeigevorrichtung der Austauschkastenbodenstütze (2) einzuschalten, und die Anzeigevorrichtung kommunizierbar mit dem mindestens einen Erfassungsschalter (24) gekoppelt ist; und/oder
(ii) der hinausragende Abschnitt der oberen Abdeckung (11) des Kastenkörpers dazu konfiguriert ist, zwei Erfassungsschalter (24) der Austauschkastenbodenstütze (2) auszulösen, und die zwei Erfassungsschalter (24) jeweils auf zwei Seiten der Austauschkastenbodenstütze (2) angeordnet sind.

6. Austauschkasten-(1)Montagestruktur nach einem der vorhergehenden Ansprüche, wobei die zweite magnetische Anziehungsvorrichtung (23) der Austauschkastenbodenstütze (2) aus einem Metallmaterial oder einem magnetischen Material hergestellt ist.

7. Austauschkasten-(1)Montagestruktur nach einem der vorhergehenden Ansprüche, wobei eine Höhe von der Bodenfläche (111) der oberen Abdeckung der oberen Abdeckung (11) des Kastenkörpers, die über die untere Abdeckung (12) des Kastenkörpers zu einem Bodenabschnitt der ersten magnetischen Anziehungsvorrichtung (15) des Austauschkastens (1) hinausragt, kleiner ist als eine Höhe von einem höchsten Abschnitt der zweiten magnetischen Anziehungsvorrichtung (23) der Austauschkastenbodenstütze (2) zu einem höchsten Abschnitt des mindestens einen Erfassungsschalters (24).

8. Verfahren zum Zusammenbauen einer Austauschkasten-(1)Montagestruktur für eine Urinanalysevorrichtung, wobei das Verfahren Folgendes umfasst:
Bereitstellen eines Austauschkastens (1), der eine erste magnetische Anziehungsvorrichtung (15) umfasst, die an einem Bodenabschnitt einer unteren Abdeckung (12) eines Kastenkörpers des Austauschkastens (1) angeordnet ist und einen hinausragenden Abschnitt umfasst, indem es einer Bodenfläche (111) einer oberen Abdeckung einer oberen Abdeckung (11) eines Kastenkörpers des Austauschkastens ermöglicht wird, über die untere Abdeckung (12) des Kastenkörpers des Austauschkastens (1) hinauszuragen;
Bereitstellen einer Austauschkastenbodenstütze (2), die eine zweite magnetische Anziehungsvorrichtung (23) umfasst, die an einem Bodenabschnitt eines Bodenstützenhohlraums (22) angeordnet ist;
Einsetzen des Austauschkastens (1) in den Bodenstützenhohlraum (22) der Austauschkastenbodenstütze (2); und
Führen des Austauschkastens (1), um durch eine Anziehung zwischen der ersten magnetischen Anziehungsvorrichtung (15) und der zweiten magnetischen Anziehungsvorrichtung (23) eine voreingestellte Position zu erreichen; und
Auslösen, durch den hinausragenden Abschnitt, mindestens eines Erfassungsschalters (24) der Austauschkastenbodenstütze (2), um ein Signal an Ort und Stelle zu erzeugen.

9. Verfahren nach Anspruch 10, ferner umfassend:
Einschalten einer Anzeigevorrichtung, die kommunizierbar mit dem mindestens einen Erfassungsschalter (24) gekoppelt ist, als Reaktion auf das durch den mindestens einen Erfassungsschalter (24) erzeugte Signal an Ort und Stelle.

## Revendications

1. Structure de montage de boîte de remplacement (1) pour un dispositif d'analyse d'urine comprenant :
une boîte de remplacement (1) comprenant :
un couvercle supérieur de corps de boîte (11) ;
un couvercle inférieur de corps de boîte (12) configuré pour être relié au couvercle supérieur de corps de boîte (11) pour former une cavité de corps de boîte (13), la cavité de corps de boîte (13) étant configurée pour loger du papier de test (14) ; et
un premier dispositif d'attraction magnétique (15) disposé au niveau d'une partie inférieure du couvercle inférieur de corps de boîte (12) ; et
un support inférieur de boîte de remplacement (2) comprenant :
un corps de support inférieur (21) ; **caractérisée par**
au moins un commutateur de détection (24) disposé au niveau du corps de support inférieur (21) et configuré pour détecter si la boîte de remplacement (1) atteint une position prédéfinie ;
une cavité de support inférieur (22) disposée dans le corps de support inférieur (21) et configurée pour recevoir la boîte de remplacement (1) ; et
un second dispositif d'attraction magnétique (23) disposé au niveau d'une partie inférieure de la cavité de support inférieur (22),
dans laquelle le premier dispositif d'attraction magnétique (15) est configuré pour être attiré pour guider la boîte de remplacement (1) pour atteindre la position prédéfinie lorsque la boîte de remplacement (1) est insérée dans le support inférieur de boîte de remplacement (2),
dans laquelle le second dispositif d'attraction magnétique (23) du support inférieur de boîte de remplacement (2) est configuré pour guider la boîte de remplacement (1) pour atteindre la position prédéfinie lorsque la boîte de remplacement (1) est insérée dans le support inférieur de boîte de remplacement (2),
dans laquelle une surface inférieure de couvercle supérieur (111) du couvercle supérieur de corps de boîte (11) fait saillie au-delà du couvercle inférieur de corps de boîte (12) pour former une partie en saillie, et
dans laquelle la partie en saillie du couvercle supérieur de corps de boîte (11) est configurée pour déclencher l'au moins un commutateur de détection (24) du support inférieur de boîte de remplacement (2) de sorte à générer un signal de mise en place en appuyant sur l'au moins un commutateur de détection (24).

2. Structure de montage de boîte de remplacement (1) selon la revendication 1, dans laquelle le premier dispositif d'attraction magnétique (15) est configuré pour être attiré vers un second dispositif d'attraction magnétique (23) du support inférieur de boîte de remplacement (2) de sorte à atteindre la position prédéfinie lorsque la boîte de remplacement (1) est insérée dans le support inférieur de boîte de remplacement (2).

3. Structure de montage de boîte de remplacement (1) selon la revendication 2, dans laquelle le premier dispositif d'attraction magnétique (15) est constitué d'un matériau magnétique ou d'un matériau métallique.

4. Boîte de remplacement (1) selon la revendication 3, dans laquelle un du premier dispositif d'attraction magnétique (15) et du second dispositif d'attraction magnétique (23) est constitué du matériau magnétique et l'autre du premier dispositif d'attraction magnétique (15) et du second dispositif d'attraction magnétique (23) est constitué du matériau métallique.

5. Structure de montage de boîte de remplacement (1) selon l'une quelconque des revendications précédentes, dans laquelle :
(i) le signal de mise en place est configuré pour allumer un dispositif d'indication du support inférieur de boîte de remplacement (2) et le dispositif d'indication est couplé en communication à l'au moins un commutateur de détection (24) ; et/ou
(ii) la partie en saillie du couvercle supérieur de corps de boîte (11) est configurée pour déclencher deux commutateurs de détection (24) du support inférieur de boîte de remplacement (2) et les deux commutateurs de détection (24) sont disposés respectivement sur deux côtés du support inférieur de boîte de remplacement (2).

6. Structure de montage de boîte de remplacement (1) selon l'une quelconque des revendications précédentes, dans laquelle le second dispositif d'attraction magnétique (23) du support inférieur de boîte de remplacement (2) est constitué d'un matériau métallique ou d'un matériau magnétique.

7. Structure de montage de boîte de remplacement (1) selon l'une quelconque des revendications précédentes, dans laquelle une hauteur depuis la surface inférieure de couvercle supérieur (111) du couvercle supérieur de corps de boîte (11) faisant saillie au-delà du couvercle inférieur de corps de boîte (12) jusqu'à une partie inférieure du premier dispositif d'attraction magnétique (15) de la boîte de remplacement (1) est inférieure à une hauteur depuis une partie supérieure du second dispositif d'attraction magnétique (23) du support inférieur de boîte de remplacement (2) jusqu'à une partie supérieure de l'au moins un commutateur de détection (24).

8. Procédé d'assemblage d'une structure de montage de boîte de remplacement (1) pour un dispositif d'analyse d'urine, le procédé comprenant :
la fourniture d'une boîte de remplacement (1) comprenant un premier dispositif d'attraction magnétique (15) disposé au niveau d'une partie inférieure d'un couvercle inférieur de corps de boîte (12) de la boîte de remplacement (1) et comprenant une partie en saillie en permettant à une surface inférieure de couvercle supérieur (111) d'un couvercle supérieur de corps de boîte (11) de la boîte de remplacement de faire saillie au-delà du couvercle inférieur de corps de boîte (12) de la boîte de remplacement (1) ;
la fourniture d'un support inférieur de boîte de remplacement (2) comprenant un second dispositif d'attraction magnétique (23) disposé au niveau d'une partie inférieure d'une cavité de support inférieur (22) ;
l'insertion de la boîte de remplacement (1) dans la cavité de support inférieur (22) du support inférieur de boîte de remplacement (2) ; et
le guidage de la boîte de remplacement (1) pour atteindre une position prédéfinie par une attraction entre le premier dispositif d'attraction magnétique (15) et le second dispositif d'attraction magnétique (23) ; et
le déclenchement, par la partie en saillie, d'au moins un commutateur de détection (24) du support inférieur de boîte de remplacement (2) pour générer un signal de mise en place.

9. Procédé selon la revendication 10, comprenant en outre :
l'allumage d'un dispositif d'indication, couplé en communication à l'au moins un commutateur de détection (24), en réponse au signal de mise en place généré par l'au moins un commutateur de détection (24).
